# EUROPÄISCHE PATENTSCHRIFT

(11) **EP 3 029 047 B1**
(45) Veröffentlichungstag und Bekanntmachung des Hinweises auf die Patenterteilung: **17.05.2017**
(21) Anmeldenummer: 14196185.4
(22) Anmeldetag: 04.12.2014
(51) Int. Cl.: C07F 9/6574, C07B 41/06, C07C 45/50, C07F 9/145

(54) **TERPHENYL-2-OXY-PHOSPHITE ALS LIGANDEN IN HYDROFORMYLIERUNGS- KATALYSATOREN**
TERPHENYL-2-OXY-PHOSPHITES AS LIGANDS IN HYDROFORMYLATION CATALYSTS
TERPHENYL-2-OXY-PHOSPHITES EN TANT QUE LIGANDS DANS DES CATALYSATEURS D'HYDROFORMYLATION

(43) Veröffentlichungstag der Anmeldung: 08.06.2016
(60) Teilanmeldung: 15185810.7
(73) Patentinhaber: Evonik Degussa GmbH, 45128 Essen (DE)
(72) Erfinder: Dyballa, Katrin Marie, 45657 Recklinghausen (DE); Franke, Robert, 45772 Marl (DE); Fridag, Dirk, 45721 Haltern am See (DE); Hess, Dieter, 45770 Marl (DE); Geilen, Frank, 45721 Haltern am See (DE); Börner, Armin, 18059 Rostock (DE); Selent, Detlef, 18059 Rostock (DE); Uhlemann, Marcus, 65835 Liederbach am Taunus (DE)

(56) Entgegenhaltungen:
- EP-A1- 2 567 949
- WO-A1-85/03702
- CN-A- 101 288 852
- RO-B1- 104 658
- HUGO TRICAS ET AL: "Bulky monophosphite ligands for ethene hydroformylation", JOURNAL OF CATALYSIS, Bd. 298, 10. Januar 2013 (2013-01-10), Seiten 198-205, XP055185769, ISSN: 0021-9517, DOI: 10.1016/j.jcat.2012.11.031
- COLBEY J ET AL: "Synthesis and application of a new bisphosphite ligand collection for asymmetric hydroformylation of allyl cyanide", THE JOURNAL OF ORGANIC CHEMISTRY, AMERICAN CHEMICAL SOCIETY, US, Bd. 69, 11. Juni 2004 (2004-06-11), Seiten 4031-4040, XP002987930, ISSN: 0022-3263, DOI: 10.1021/JO040128P

## Beschreibung

Die Erfindung betrifft Monophosphite, die ein Diphenylphenol aufweisen. Des Weiteren deren Verwendung als Liganden in der Hydroformylierung.

Die Reaktionen zwischen Olefinverbindungen, Kohlenmonoxid und Wasserstoff in Gegenwart eines Katalysators zu den um ein C-Atom reicheren Aldehyden ist als Hydroformylierung bzw. Oxierung bekannt. Als Katalysatoren in diesen Reaktionen werden häufig Verbindungen der Übergangsmetalle der VIII. Gruppe des Periodensystems der Elemente verwendet. Bekannte Liganden sind beispielsweise Verbindungen aus den Klassen der Phosphine, Phosphite und Phosphonite mit jeweils dreiwertigen Phosphor PIII. Eine gute Übersicht über den Stand der Hydroformylierung von Olefinen findet sich in B. CORNILS, W. A. HERRMANN, "Applied Homogeneous Catalysis with Organometallic Compounds", Vol. 1 & 2, VCH, Weinheim, New York, 1996 bzw. R. Franke, D. Selent, A. Börner, "Applied Hydroformylation", Chem. Rev., 2012, DOI:10.1021/cr3001803.

Jede katalytisch aktive Zusammensetzung hat ihre spezifischen Vorzüge. Je nach Einsatzstoff und Zielprodukt kommen daher unterschiedliche katalytisch aktive Zusammensetzungen zum Einsatz.

In EP 0 155 508 B1 werden unterschiedliche Monophosphite aufgezeigt. Die abgebildeten Monophosphite weisen alle eine symmetrische Biphenyleinheit auf, d.h. die beiden Phenylringe lassen sich durch eine zwischen ihnen liegende Spiegelebene aufeinander abbilden. Die einzige Ausnahme stellt die Verbindung 16 in Tabelle 6 (Seite 58) dar. Dieser Ligand wurde nur zu Vergleichsversuchen verwendet, und weist mit einem Wert von 0,22 eine niedrige Umsatzrate auf.

In WO 85/03702 wird ein Verfahren zur Carbonilierung beschrieben. Das Verfahren wird mit einem Katalysatorkomplex betrieben, wobei der Komplex Diorganophosphitliganden aufweist.

In HUGO TRICAS ET AL, "Bulky monophosphite ligands for ethene hydroformylation", JOURNAL OF CATALYSIS, (20130110), Vol. 298, doi:10.1016/j.jcat.2012.11.031, ISSN 0021-9517, Seiten 198 - 205, wird die Hydroformylierung von Olefinen beschrieben. Es wurden unterschiedliche Liganden als Katalysator getestet.

In COLBEY J ET AL, "Synthesis and application of a new bisphosphite ligand collection for asymmetric hydroformylation of allyl cyanide", THE JOURNAL OF ORGANIC CHEMISTRY, AMERICAN CHEMICAL SOCIETY, US, (20040611), vol. 69, doi:10.1021/JO040128P, ISSN 0022-3263, Seiten 4031 - 4040 wird die Synthese von Bisphosphitliganden beschrieben. Diese wurden dann in der asymmetrischen Hydroformylierung von Allylcyaniden eingesetzt.

Der Erfindung lag die Aufgabe zugrunde, Monophosphite bereitzustellen, welche gegenüber den bekannten Monophosphiten vorteilhafte Eigenschaften in der Hydroformylierung aufweisen. Insbesondere bestand die Aufgabe darin, neue Liganden bereitzustellen, deren Einsatz gegenüber strukturverwandten Monophosphiten, welche ebenfalls eine Biphenoleinheiten aufweisen, zu einer verbesserten Ausbeute führen. Die verbesserte Ausbeute sollte bei zumindest einem Olefin realisiert werden.

Die Aufgabe wird gelöst durch eine Verbindung nach Anspruch 1.

Verbindung, welche die allgemeine Struktur I aufweist: 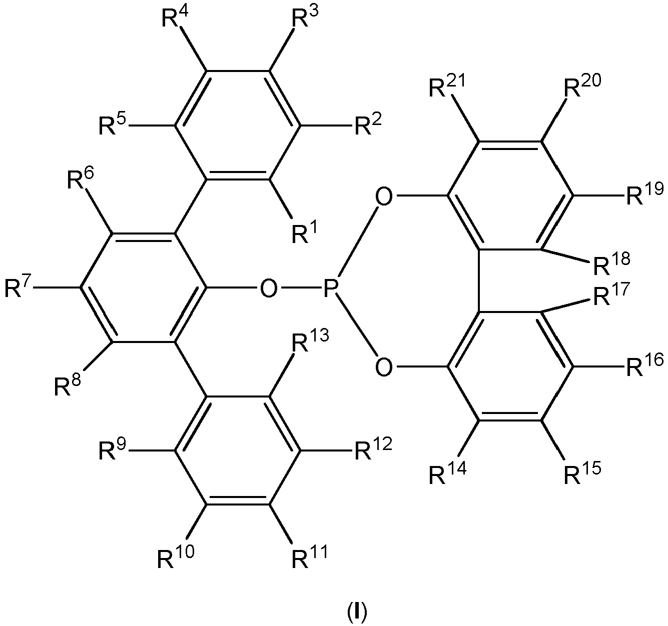 wobei
R¹, R², R³, R⁴, R⁵, R⁶, R⁷, R⁸, R⁹, R¹⁰, R¹¹, R¹², R¹³ ausgewählt sind aus:
   -H, -(C₁-C₁₂)-Alkyl, -O-(C₁-C₁₂)Alkyl, -O-(C₆-C₂₀)-Aryl, -(C₆-C₂₀)-Aryl, -S-Alkyl, -S-Aryl, Halogen, COO-(C₁-C₁₂)-Alkyl, CONH-(C₁-C₁₂)-Alkyl, , -CO-(C₁-C₁₂)-Alkyl, -CO-(C₆-C₂₀)-Aryl, -COOH, -OH, -SO₃H, -CN, -NH₂, -N[(C₁-C₁₂)-Alkyl]₂;
R¹⁴, R¹⁵, R¹⁶, R¹⁷, R¹⁸, R¹⁹, R²⁰, R²¹ ausgewählt sind aus:
   -H, -(C₁-C₁₂)-Alkyl, -O-(C₁-C₁₂)-Alkyl, -O-(C₆-C₂₀)-Aryl, -(C₆-C₂₀)-Aryl, -S-Alkyl, -S-Aryl, Halogen, COO-(C₁-C₁₂)-Alkyl, CONH-(C₁-C₁₂)-Alkyl, -CO-(C₁-C₁₂)-Alkyl, -CO-(C₆-C₂₀)-Aryl, -COOH, -OH, - SO₃H, -NH₂, -N[(C₁-C₁₂)-Alkyl]₂;
   wobei die genannten Alkyl- und Arylgruppen wie folgt substituiert sein können:
   substituierte -(C₁-C₁₂)-Alkylgruppen und substituierte -(C₁-C₁₂)-Alkoxygruppen können in Abhängigkeit von ihrer Kettenlänge, einen oder mehrere Substituenten aufweisen; die Substituenten sind unabhängig voneinander ausgewählt unter -(C₃-C₁₂)-Cycloalkyl, -(C₃-C₁₂)-Heterocycloalkyl, -(C₆-C₂₀)-Aryl, Fluor, Chlor, Cyano, Formyl, Acyl oder Alkoxycarbonyl,
   substituierte -(C₆-C₂₀)-Arylgruppen und -(C₆-C₂₀)-Aryl-(C₆-C₂₀)-Arylgruppen können, in Abhängigkeit von der Ringgröße, einen oder mehrere Substituenten aufweisen; diese Substituenten sind unabhängig voneinander ausgewählt unter -H, -(C₁-C₁₂)-Alkyl, -O-(C₁-C₁₂)-Alkyl, -O-(C₆-C₂₀)-Aryl, -(C₆-C₂₀)-Aryl, -Halogen, -COO-(C₁-C₁₂)-Alkyl, -CONH-(C₁-C₁₂)-Alkyl, -(C₆-C₂₀)-Aryl-CON[(C₁-C₁₂)-Alkyl]₂, -CO-(C₁-C₁₂)-Alkyl, -CO-(C₆-C₂₀)-Aryl, -COOH, -OH, -SO₃H; - SO₃Na, -NO₂, -CN, -NH₂, -N[(C₁-C₁₂)-Alkyl]₂,
   und die beiden Reste mindestens eines der vier folgenden Restepaare nicht für den gleichen Rest stehen: R¹⁴ und R²¹, R¹⁵ und R²⁰, R¹⁶ und R¹⁹, R¹⁷ und R¹⁸.

Durch das Merkmal "und die beiden Reste mindestens eines der vier folgenden Restepaare nicht für den gleichen Rest stehen: R¹⁴ und R²¹, R¹⁵ und R²⁰, R¹⁶ und R¹⁹, R¹⁷ und R¹⁸" wird zum Ausdruck gebracht, dass es sich bei mindestens einem der beiden Biphenol-Flügel-Baustein um ein unsymmetrisches Biphenol handelt. Es besteht auch die Möglichkeit, dass beide Biphenol-Flügel-Baustein gleichzeitig unsymmetrisch sind. Bei unsymmetrischen Biphenolen lassen sich die beiden Aromaten nicht durch eine zwischen ihnen liegende Spiegelebene aufeinander abbilden.

Zugelassen sind folgende Restepaarungen, wie beispielsweise:
R¹⁴ ungleich R²¹, R¹⁵ gleich R²⁰, R¹⁶ gleich R¹⁹, R¹⁷ gleich R¹⁸,
R¹⁴ gleich R²¹, R¹⁵ gleich R²⁰, R¹⁶ ungleich R¹⁹, R¹⁷ gleich R¹⁸.

Ober aber auch Restepaarungen bei denen mehr als nur ein Paar ungleich ist, wie beispielsweise:
R¹⁴ ungleich R²¹, R¹⁵ gleich R²⁰, R¹⁶ ungleich R¹⁹, R¹⁷ gleich R¹⁸;
R¹⁴ ungleich R²¹, R¹⁵ ungleich R²⁰, R¹⁶ ungleich R¹⁹, R¹⁷ gleich R¹⁸.

Ausgeschlossen wird lediglich der Fall, bei dem alle vier Restepaare jeweils paarweise für den gleichen Rest stehen:
R¹⁴ gleich R²¹, R¹⁵ gleich R²⁰, R¹⁶ gleich R¹⁹, R¹⁷ gleich R¹⁸.
Hierbei würde es sich um ein symmetrisches Biphenol handeln.

(C₁-C₁₂)-Alkyl und O-(C₁-C₁₂)-Alkyl können jeweils unsubstituiert oder durch einen oder mehrere gleiche oder verschiedene Reste substituiert sein, die ausgewählt sind unter (C₃-C₁₂)-Cycloalkyl, (C₃-C₁₂)-Heterocycloalkyl, (C₆-C₂₀)-Aryl, Fluor, Chlor, Cyano, Formyl, Acyl oder Alkoxycarbonyl.

(C₆-C₂₀)-Aryl und -(C₆-C₂₀)-Aryl-(C₆-C₂₀)-Aryl- können jeweils unsubstituiert oder durch einen oder mehrere gleiche oder verschiedene Reste substituiert sein, die ausgewählt sind unter -H, -(C₁-C₁₂)-Alkyl, -O-(C₁-C₁₂)Alkyl, -O-(C₆-C₂₀)-Aryl, -(C₆-C₂₀)-Aryl, -Halogen (wie Cl, F, Br, I), - COO-(C₁-C₁₂)-Alkyl, -CONH-(C₁-C₁₂)-Alkyl, -(C₆-C₂₀)-Aryl-CON[(C₁-C₁₂)-Alkyl]₂, -CO-(C₁-C₁₂)-Alkyl, -CO-(C₆-C₂₀)-Aryl, -COOH, -OH, -SO₃H -SO₃Na -NO₂, -CN, -NH₂, -N[(C₁-C₁₂)-Alkyl]₂.

Im Rahmen der Erfindung umfasst der Ausdruck -(C₁-C₁₂)-Alkyl geradkettige und verzweigte Alkylgruppen. Vorzugsweise handelt es sich dabei um unsubstituierte geradkettige oder verzweigte -(C₁-C₈)-Alkyl- und ganz bevorzugt -(C₁-C₆)-Alkylgruppen. Beispiele für -(C₁-C₁₂)-Alkylgruppen sind insbesondere Methyl, Ethyl, Propyl, Isopropyl, n-Butyl, iso-Butyl, sec.-Butyl, tert.-Butyl, n-Pentyl, 2-Pentyl, 2-Methylbutyl-, 3-Methylbutyl-, 1,2-Dimethylpropyl-, 1,1-Dimethylpropyl, 2,2-Dimethylpropyl-, 1-Ethylpropyl-, n-Hexyl-, 2-Hexyl-, 2-Methylpentyl-, 3-Methylpentyl-, 4-Methylpentyl-, 1,1-Dimethylbutyl-, 1,2-Diemthylbutyl-, 2,2-Dimethylbutyl-, 1,3-Dimethylbutyl-, 2,3-Dimethylbutyl-, 3,3-Dimethylbutyl-, 1,1,2-Trimethylpropyl-, 1,2,2-Trimethylpropyl-, 1-Ethylbutyl-, 1-Ethyl-2-methylpropyl-, n-Heptyl-, 2-Heptyl-, 3-Heptyl-, 2-Ethylpentyl-, 1-Propylbutyl-, n-Octyl-, 2-Ethylhexyl-, 2-Propylheptyl-, Nonyl-, Decyl.

Die Erläuterungen zum Ausdruck -(C₁-C₁₂)-Alkyl gelten auch für die Alkylgruppen in -O-(C₁-C₁₂)-Alkyl, also in -(C₁-C₁₂)-Alkoxy. Vorzugsweise handelt es sich dabei um unsubstituierte geradkettige oder verzweigte -(C₁-C₆)-Alkoxygruppen.

Substituierte -(C₁-C₁₂)-Alkylgruppen und substituierte -(C₁-C₁₂)-Alkoxygruppen können in Abhängigkeit von ihrer Kettenlänge, einen oder mehrere Substituenten aufweisen. Die Substituenten sind vorzugsweise unabhängig voneinander ausgewählt unter-(C₃-C₁₂)-Cycloalkyl, -(C₃-C₁₂)-Heterocycloalkyl, -(C₆-C₂₀)-Aryl, Fluor, Chlor, Cyano, Formyl, Acyl oder Alkoxycarbonyl.

Der Ausdruck -(C₃-C₁₂)-Cycloalkyl umfasst im Sinne der vorliegenden Erfindung mono-, bi- oder tricyclische Kohlenwasserstoffreste mit 3 bis 12, insbesondere 5 bis 12 Kohlenstoffatomen. Dazu zählen Cyclopropyl-, Cyclobutyl-, Cyclopentyl-, Cyclohexyl-, Cycloheptyl-, Cyclooctyl-, Cyclododecyl-, Cyclopentadecyl-, Norbonyl- oder Adamantyl.
Ein Beispiel für ein substituiertes Cycloalkyl wäre Menthyl.

Der Ausdruck -(C₃-C₁₂)-Heterocycloalkylgruppen umfasst im Sinne der vorliegenden Erfindung nichtaromatische, gesättigte oder teilweise ungesättigte cycloaliphatische Gruppen mit 3 bis 12, insbesondere 5 bis 12, Kohlenstoffatomen. Die -(C₃-C₁₂)-Heterocycloalkylgruppen weisen vorzugsweise 3 bis 8, besonders bevorzugt 5 oder 6, Ringatome auf. In den Heterocycloalkylgruppen sind im Unterschied zu den Cycloalkylgruppen 1, 2, 3 oder 4 der Ringkohlenstoffatome durch Heteroatome oder heteroatomhaltige Gruppen ersetzt. Die Heteroatome oder die heteroatomhaltige Gruppen sind vorzugsweise ausgewählt unter -O-, -S-, -N-, -N(=O)-, -C(=O)- oder -S(=O)-. Beispiele für -(C₃-C₁₂)-Heterocycloalkylgruppen Tetrahydrothiophenyl, Tetrhydrofuryl, Tetrahydropyranyl und Dioxanyl.

Der Ausdruck -(C₆-C₂₀)-Aryl und -(C₆-C₂₀)-Aryl-(C₆-C₂₀)-Aryl- umfasst im Sinne der vorliegenden Erfindung mono- oder polycyclische aromatische Kohlenwasserstoffreste. Diese weisen 6 bis 20 Ringatome, besonders bevorzugt 6 bis 14 Ringatome, insbesondere 6 bis 10 Ringatome, auf. Aryl steht vorzugsweise für -(C₆-C₁₀)-Aryl und -(C₆-C₁₀)-Aryl-(C₆-C₁₀)-Aryl-. Aryl steht insbesondere für Phenyl, Naphthyl, Indenyl, Fluorenyl, Anthracenyl, Phenanthrenyl, Naphthacenyl, Chrysenyl, Pyrenyl, Coronenyl. Insbesondere steht Aryl für Phenyl, Naphthyl und Antracenyl.

Substituierte -(C₆-C₂₀)-Arylgruppen und -(C₆-C₂₀)-Aryl-(C₆-C₂₀)-Arylgruppen können, in Abhängigkeit von der Ringgröße, einen oder mehrere (z.B. 1, 2, 3, 4 oder 5) Substituenten aufweisen. Diese Substituenten sind vorzugsweise unabhängig voneinander ausgewählt unter - H, -(C₁-C₁₂)-Alkyl, -O-(C₁-C₁₂)-Alkyl, -O-(C₆-C₂₀)-Aryl, -(C₆-C₂₀)-Aryl, -Halogen (wie Cl, F, Br, I), - COO-(C₁-C₁₂)-Alkyl, -CONH-(C₁-C₁₂)-Alkyl, -(C₆-C₂₀)-Aryl-CON[(C₁-C₁₂)-Alkyl]₂, -CO-(C₁-C₁₂)-Alkyl, -CO-(C₆-C₂₀)-Aryl, -COOH, -OH, -SO₃H -SO₃Na -NO₂, -CN, -NH₂, -N[(C₁-C₁₂)-Alkyl]₂.
Substituierte -(C₆-C₂₀)-Arylgruppen und -(C₆-C₂₀)-Aryl-(C₆-C₂₀)-Arylgruppen sind vorzugsweise substituierte -(C₆-C₁₀)-Arylgruppen und -(C₆-C₁₀)-Aryl-(C₆-C₁₀)-Arylgruppen, insbesondere substituiertes Phenyl oder substituiertes Naphthyl oder substituiertes Anthracenyl. Substituierte - (C₆-C₂₀)-Arylgruppen tragen vorzugsweise eine oder mehrere z.B. 1, 2, 3, 4 oder 5 Substituenten, ausgewählt unter -(C₁-C₁₂)-Alkylgruppen, -(C₁-C₁₂)-Alkoxygruppen.

In einer Ausführungsform sind R¹, R², R³, R⁴, R⁵, R⁶, R⁷, R⁸, R⁹, R¹⁰, R¹¹, R¹², R¹³ ausgewählt aus:
-H, -(C₁-C₁₂)-Alkyl, -O-(C₁-C₁₂)-Alkyl, -O-(C₆-C₂₀)-Aryl, -S-Alkyl, -S-Aryl.

In einer Ausführungsform sind R¹⁴, R¹⁵, R¹⁶, R¹⁷, R¹⁸, R¹⁸, R²⁰, R²¹ ausgewählt aus:
-H, -(C₁-C₁₂)-Alkyl, -O-(C₁-C₁₂)-Alkyl, -O-(C₆-C₂₀)-Aryl, -S-Alkyl, -S-Aryl.

In einer Ausführungsform sind R¹, R², R³, R⁴, R⁵, R⁶, R⁷, R⁸, R⁹, R¹⁰, R¹¹, R¹², R¹³ ausgewählt aus:
-H, -(C₁-C₁₂)-Alkyl, -O-(C₁-C₁₂)-Alkyl, -O-(C₆-C₂₀)-Aryl.

In einer Ausführungsform sind R¹⁴, R¹⁵, R¹⁸, R¹⁷, R¹⁸, R¹⁸, R²⁰, R²¹ ausgewählt aus:
-H, -(C₁-C₁₂)-Alkyl, -O-(C₁-C₁₂)-Alkyl, -O-(C₆-C₂₀)-Aryl.

In einer Ausführungsform sind R¹, R², R³, R⁴, R⁵, R⁶, R⁷, R⁸, R⁹, R¹⁰, R¹¹, R¹², R¹³ ausgewählt aus:
-H, -(C₁-C₁₂)-Alkyl, -O-(C₁-C₁₂)-Alkyl.

In einer Ausführungsform sind R¹⁴, R¹⁵, R¹⁸, R¹⁷, R¹⁸, R¹⁹, R²⁰, R²¹ ausgewählt aus:
-H, -(C₁-C₁₂)-Alkyl, -O-(C₁-C₁₂)-Alkyl.

In einer Ausführungsform stehen R¹⁴ und R²¹ nicht für den gleichen Rest.

In einer Ausführungsform stehen R¹⁵ und R²⁰ nicht für den gleichen Rest.

In einer Ausführungsform stehen R¹⁶ und R¹⁹ nicht für den gleichen Rest.

In einer Ausführungsform stehen R¹⁷ und R¹⁸ nicht für den gleichen Rest.

In einer Ausführungsform weist die Verbindung eine der folgenden Strukturen (5) bis (9) auf:

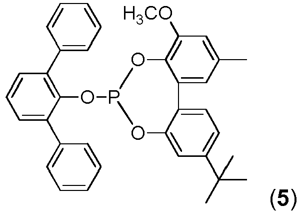

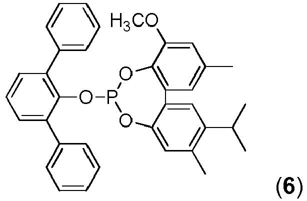

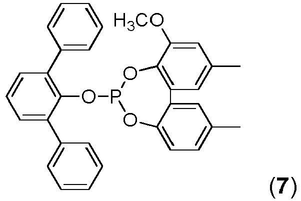

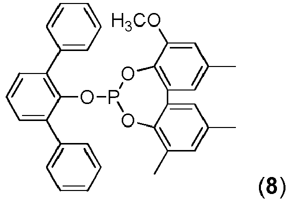

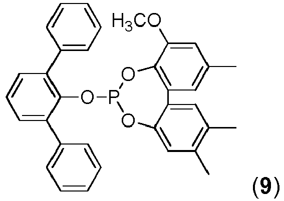

Neben den Verbindungen wird auch ein Komplex beansprucht, welcher diese Verbindungen umfasst.

Komplex umfassend:
- eine zuvor beschriebene Verbindung,
- ein Metallatom ausgewählt aus: Rh, Ru, Co, Ir.

In einer bevorzugten Ausführungsform ist das Metall Rh.

Siehe hierzu R. Franke, D. Selent, A. Börner, "Applied Hydroformylation", Chem. Rev., 2012, DOI:10.1021/cr3001803; S. 5688 Schema 12 "General Method for the Preparation of a P-Modified Rh precatalyst" und darin zitierte Literaturstellen sowie P. W. N. M. van Leeuwen, in Rhodium Catalyzed Hydroformylation, P. W. N. M. van Leeuwen, C. Claver (Hrsg.), Kluwer, Dordrecht, 2000 unter anderem S. 48 ff, S.233 ff. und darin zitierte Literaturstellen sowie K.D. Wiese und D. Obst in Top. Organomet. Chem. 2006, 18, 1-13; Springer Verlag Berlin Heidelberg 2006 S. 6 ff sowie darin zitierte Literaturstellen.

Des Weiteren wird die Verwendung der Verbindung als Ligand in einem Ligand-Metall-Komplex zur Katalyse einer Hydroformylierungsreaktion beansprucht.

Verwendung einer zuvor beschriebenen Verbindung in einem Ligand-Metall-Komplex zur Katalyse einer Hydroformylierungsreaktion.

Das Verfahren bei dem die Verbindung als Ligand in einem Ligand-Metall-Komplex zur Umsetzung eines Olefins zu einem Aldehyd eingesetzt wird, wird ebenfalls beansprucht.

Verfahren umfassend die Verfahrensschritte:
a) Vorlegen eines Olefins,
b) Zugabe eines zuvor beschriebenen Komplexes,
   oder einer zuvor beschriebenen Verbindung und einer Substanz, welche ein Metallatom ausgewählt aus: Rh, Ru, Co, Ir aufweist,
c) Zuführen von H₂ und CO,
d) Erwärmen des Reaktionsgemisches, wobei das Olefin zu einem Aldehyd umgesetzt wird.

Hierbei können die Verfahrensschritte a) bis d) in beliebiger Reihenfolge erfolgen.

In einer bevorzugten Variante des Verfahrens ist das Metallatom Rh.

Es kann hierbei auch ein Überschuss an Liganden verwendet werden und nicht zwangsläufig jeder Ligand liegt gebunden in Form eines Ligand-Metall-Komplexes vor, sondern ist als freier Ligand im Reaktionsgemisch enthalten.

Die Reaktion wird bei üblichen Bedingungen durchgeführt.

Bevorzugt sind eine Temperatur von 80 °C bis 200 °C und ein Druck von 1 bar bis 300 bar. Besonders bevorzugt sind eine Temperatur von 100 °C bis 160 °C und ein Druck von 15 bar bis 250 bar.

Die Edukte für die Hydroformylierung gemäß dem Verfahren der Erfindung sind Olefine oder Gemische von Olefinen, insbesondere Monoolefine mit 2 bis 24, bevorzugt 3 bis 16, besonders bevorzugt 3 bis 12 Kohlenstoffatomen mit end- oder innenständigen C-C-Doppelbindungen, wie z.B. 1-Propen, 1- oder 2-Penten, 2-Methyl-1-buten, 2-Methyl-2-buten, 3-Methyl-1-buten, 1-, 2-oder 3,-Hexen, das bei der Dimerisierung von Propen anfallende C₆-Olefingemisch (Dipropen), Heptene, 2- oder 3-Methyl-1-hexene, Octene, 2-Methylheptene, 3-Methylheptene, 5-Methyl-2-hepten, 6-Methyl-2-hepten, 2-Ethyl-1-hexen, das bei der Dimerisierung von Butenen anfallende C₈-Olefingemisch (Dibuten), Nonene, 2- oder 3-Methyloctene, das bei der Trimerisierung von Propen anfallende C₉-Olefingemisch (Tripropen), Decene, 2-Ethyl-1-octen, Dodecene, das bei der Tetramerisierung oder der Trimerisierung von Butenen anfallende C₁₂-Olefingemisch (Tetrapropen oder Tributen), Tetradecene, Hexadecene, das bei der Tetramerisierung von Butenen anfallende C₁₆-Olefingemisch (Tetrabutan) sowie durch Cooligomerisierung von Olefinen mit unterschiedlicher Anzahl von Kohlenstoffatomen (bevorzugt 2 bis 4) hergestellte Olefingemische.

Im Folgenden wird die Erfindung anhand von Ausführungsbeispielen näher erläutert.

### Allgemeine Arbeitsvorschriften

Alle nachfolgenden Präparationen wurden mit Standard-Schlenk-Technik unter Schutzgas durchgeführt. Die Lösungsmittel wurden vor Gebrauch über geeigneten Trocknungsmitteln getrocknet (Purification of Laboratory Chemicals, W. L. F. Armarego (Autor), Christina Chai (Autor), Butterworth Heinemann (Elsevier), 6. Auflage, Oxford 2009).
Phosphortrichlorid (Aldrich) wurde vor dem Einsatz unter Argon destilliert. Alle präparativen Arbeiten erfolgten in ausgeheizten Gefäßen. Die Charakterisierung der Produkte erfolgte mittels NMR-Spektroskopie. Chemische Verschiebungen (δ) werden in ppm angegeben. Die Referenzierung der ³¹P-NMR-Signale erfolgte gemäß: SR_{31P} = SR_{1H} * (BF_{31P} / BF_{1H}) = SR_{1H} * 0,4048. (Robin K. Harris, Edwin D. Becker, Sonia M. Cabral de Menezes, Robin Goodfellow, and Pierre Granger, Pure Appl. Chem., 2001, 73, 1795-1818; Robin K. Harris, Edwin D. Becker, Sonia M. Cabral de Menezes, Pierre Granger, Roy E. Hoffman and Kurt W. Zilm, Pure Appl. Chem., 2008, 80, 59-84).
Die Aufnahme von Kernresonanzspektren erfolgte an Bruker Avance 300 bzw. Bruker Avance 400, die gaschromatografische Analyse an Agilent GC 7890A, die Elementaranalyse an Leco TruSpec CHNS und Varian ICP-OES 715, und die ESI-TOF Massenspektrometrie an Thermo Electron Finnigan MAT 95-XP und Agilent 6890 N/5973 Geräten.

### Synthese

### Biphenole

Die Synthese der Biphenole erfolgt analog zu DE102013203865 und DE102013203867.

### 2,6-Diphenylphenoxydichlorphosphin

### (Vorstufe)

Chlorophosphite lassen sich durch Zusatz von Phosphortrichlorit in Gegenwart einer Base herstellen. Die Synthese des 2,6-Diphenylphenoxydichlorphosphin erfolgte gemäß "Phosphorous(III) Ligands in Homogeneous Catalysis - Design and Synthesis" von Paul C.J. Kamer und Piet W.N.M. van Leeuwen; John Wiley and Sons, 2012; unter anderem S. 94 ff. und darin zitierte Literaturstellen.

### 6-([1,1':3',1"-Terphenyl]-2'-yloxy)dibenzo[d,f][1,3,2]dioxaphosphepin.

### (Vergleichsverbindung)

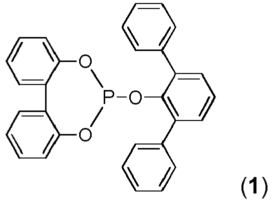

Eine Lösung von 2,6-Diphenylphenol (0,491 g; 1,99 mmol) in Toluol (9 ml) wurde mit Triethylamin (1,851 g; 18,293 mmol) versetzt und die erhaltene Mischung tropfenweise bei 0°C zu einer Lösung von 6-Chlorodibenzo[*d*,*f*][1,3,2]dioxaphosphepin (0,500 g; 1,99 mmol) in Toluol (9 ml) gegeben. Die Reaktionsmischung wurde über Nacht gerührt und dann filtriert. Das Filtrat wurde im Vakuum bis zur Trockne eingeengt und der erhaltene Rückstand aus heißem Toluol (3 ml) umkristallisiert. Ausbeute: 0,536 g (1,164 mmol; 58 %).
Elementaranalyse (berechnet für C₃₀H₂₁O₃P = 460,44 g/ mol) C 78,30 (78,25); H 4,72 (4,60) %.
³¹P-NMR (CD₂Cl₂): 146,0 ppm.
¹H-NMR (CD₂Cl₂): 6,12 (m, 2 H); 7,21 (m, 4 H); 7,35-7,40 (m, 3 H); 7,43-7,46 (m, 2 H); 7,52-7,57 (m, 6 H); 7,59-7,64 (m, 4 H) ppm.
¹³C-NMR (CD₂Cl₂): 122,5; 125,2; 125,5; 127,9; 128,9; 129,5; 129,8; 131,1; 131,2; 136,4; 136,5; 138,9; 146,5 (d, *J*_{CP}= 8,5 Hz); 149,0 (d, *J*_{CP}= 4,7 Hz) ppm.
ESI-TOF/HRMS: m/e 461,13018 (M+H)⁺.

### 6-([1,1':3',1"-Terphenyl]-2'-yloxy)-4,8-di-tert-butyl-2,10-dimethoxydibenzo[d,f][1,3,2]dioxaphosphepin.

### (Vergleichsverbindung)

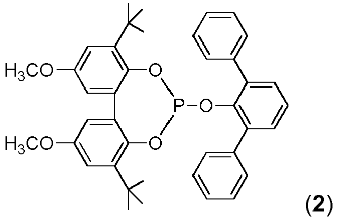

Eine Lösung von 2,6-Diphenylphenol (0,411 g; 1,65 mmol) in Toluol (8 ml) wurde mit Triethylamin (1,529 g; 15,11 mmol) versetzt und die erhaltene Mischung tropfenweise bei 0°C zu einer Lösung von 4,8-Di-*tert*-butyl-6-chloro-2,10-dimethoxydibenzo[*d*,*f*][1,3,2]dioxaphosphepin (0,697 g; 1,65 mmol) in Toluol (6 ml) gegeben. Die Reaktionsmischung wurde über Nacht bei Raumtemperatur und für 5 h bei 70 °C gerührt. Dann wurde die Mischung filtriert und das Filtrat im Vakuum bis zur Trockne eingeengt. Der erhaltene Rückstand wurde aus Hexan (4 ml) umkristallisiert. Ausbeute: 0,417 g (0,659 mmol; 40 %).
Elementaranalyse (berechnet für C₄₀H₄₁O₅P = 632,70 g/ mol) C 75,95 (75,93); H 6,52 (6,53); P 5,01 (5,00) %.
³¹P-NMR (CD₂Cl₂): 140,9 ppm.
¹H-NMR (CD₂Cl₂): 1,37 (s, 18 H); 3,84 (s, 6 H); 6,51 (d, ⁵*J*_{HH=} 3,1 Hz, 2 H, Hₐᵣₒₘ); 6,89-7,95 (m, br, 15 H, Hₐᵣₒₘ) ppm.
¹³C-NMR (CD₂Cl₂): 31,1; 35,5; 55,9; 113,0; 114,5; 125,2; 126,5-131,0 (broad, overlapping signals); 133,8 (d, *J*_{CP}= 4,0 Hz); 141,5 (d, *J*_{CP}= 6,3 Hz); 142,6; 144,8; 156,1 ppm. ESI-TOF/HRMS: m/e 633,27654 (M+H)⁺.

### 6-([1,1':3',1"-Terphenyl]-2'-yloxy)-2,4,8,10-tetra-tert-butyldibenzo[d,f][1,3,2]dioxaphosphepin.

### (Vergleichsverbindung)

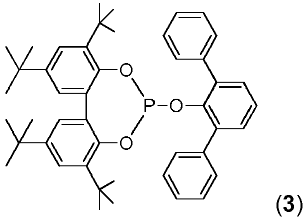

Eine Lösung von 3,3',5,5'-Tetra-*tert*-butyl-[1,1'-biphenyl]-2,2'-diol (0,616 g; 1,50 mmol) in Toluol (13 ml) wurde mit Triethylamin (1,390 g; 13,74 mmol) versetzt und auf 0 °C gekühlt. Zu dieser Mischung wurde eine Lösung von (2,6-Diphenylphenoxy)dichlorophosphin (0,521 g; 1,50 mmol) in Toluol (3 ml) tropfenweise zugegeben. Die Reaktionsmischung wurde über Nacht gerührt und filtriert. Das Filtrat wurde im Vakuum bis zur Trockne eingeengt, der erhaltene Rückstand in Dichlormethan (3 ml) aufgenommen und über Kieselgel filtriert. Das Filtrat wurde im Vakuum eingeengt und der erhaltene Feststoff bei 50°C / 0,1 mbar getrocknet. Ausbeute: 0,955 g (1,394 mmol; 93 %).
Elementaranalyse (berechnet für C₄₆H₅₃O₃P = 684,90 g/ mol) C 80,60 (80,67); H 7,71 (7,80); P 4,36 (4,52) %.
³¹P-NMR (CD₂Cl₂): 138,7 ppm.
¹H-NMR (CD₂Cl₂): 1,29-1,46 (m, br, 36 H, C(C*H*₃)₃); 6,76-7,13 (m, br, 4 H, Hₐᵣₒₘ); 7,30-7,34 (m, 1 H, Hₐᵣₒₘ); 7,34-7,40 (m, 2 H, Hₐᵣₒₘ); 7,40-7,87 (m, br, 10 H, Hₐᵣₒₘ) ppm.
¹³C-NMR (CD₂Cl₂): 31,3; 31,3; 31,7; 34,9; 35,6; 124,6; 125,2; 125,7; 126,7; 128,6; 129,4; 133,0; 137,7; 138,4; 140,5; 143,5; 144,9; 145,5; 146,9; 150,2; zusätzliche breite Signale zwischen 127 und 131 ppm.
ESI-TOF/HRMS: m/e 685,38089 (M+H)⁺.

### 6-([1,1':3',1"-Terphenyl]-2'-yloxy)-9-(tert-butyl)-4-methoxy-2-methyldibenzo[d,f][1,3,2]dioxaphosphepin.

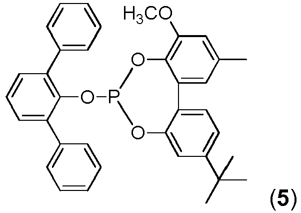

Eine gerührte Lösung von 4'-(*tert*-Butyl)-3-methoxy-5-methyl-[1,1'-biphenyl]-2,2'-diol (0,479 g; 1,67 mmol) in Toluol (12 ml) wurde mit Triethylamin (1,550 g; 15,32 mmol) versetzt und auf 0°C gekühlt. Eine Lösung von 2,6-Diphenylphenoxydichlorphosphin (0,581 g; 1,673 mmol) in Toluol (3 ml) wurde tropfenweise zugegeben. Die Reaktionsmischung wurde über Nacht gerührt und dann filtriert. Das Filtrat wurde im Vakuum bis zur Trockne eingeengt und der erhaltene Feststoff bei 50°C /0,1 mbar getrocknet. Ausbeute: 0,890 g (1,588 mmol; 96 %). Elementaranalyse (berechnet für C₃₆H₃₃O₄P = 560,63 g/ mol) C 77,07 (77,13); H 5,96 (5,93); P 5,62 (5,52) %.
³¹P-NMR (CD₂Cl₂): 144,5 ppm.
¹H-NMR (CD₂Cl₂): 41 (s, 9 H); 3,82 (s, 3 H); 6,81 (m, 3 H, Hₐᵣₒₘ); 7,21-7,64 (m, 15 H, Hₐᵣₒₘ) ppm.
¹³C-NMR (CD₂Cl₂): 21,7; 31,5; 35,0; 56,4; 112,9; 119,3; 121,5; 122,3; 125,0; 125,7; 127,7; 128,4; 128,6; 129,4; 130,7; 131,1; 132,3 (d, *J*_{CP}= 3,8 Hz); 135,5; 136,3 (d, *J*_{CP}= 3,5 Hz); 138,9; 146,9 (d, *J*_{CP}= 4,0 Hz); 149,8 (d, *J*_{CP}= 8,1 Hz); 151,9; 153,1 ppm.
ESI-TOF/HRMS: m/e 561,21916 (M+H)⁺.

### 6-([1,1':3',1"-Terphenyl]-2'-yloxy)-2-isopropyl-8-methoxy-3,10-dimethyldibenzo[d,f][1,3,2]dioxaphosphepin.

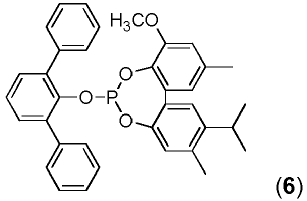

Eine Lösung von 5'-Isopropyl-3-methoxy-4',5-dimethyl-[1,1'-biphenyl]-2,2'-diol (0,478 g; 1,670 mmol) in Toluol (12 ml) wurde mit Triethylamin (1,548 g; 15,296 mmol) versetzt. Die Mischung wurde auf 0 °C gekühlt und eine Lösung von 2,6-Diphenylphenoxydichlorphosphin (0,580 g; 1,670 mmol) in Toluol (3 ml) tropfenweise zugegeben. Die Reaktionsmischung wurde über Nacht gerührt und dann filtriert. Das Filtrat wurde im Vakuum bis zur Trockne eingeengt, der erhaltene Feststoff bei 50 °C getrocknet und durch Säulenchromatografie gereinigt (Dichlormethan, R*_{f}* = 0,8). Ausbeute: 0,642 g (1,144 mmol; 69 %).
Elementaranalyse (berechnet für C₃₆H₃₃O₄P = 560,63 g/ mol) C 77,07 (77,13); H 5,73 (5,93); P 5,56 (5,52) %.
³¹P-NMR (CD₂Cl₂): 143,7 ppm.
¹H-NMR (CD₂Cl₂): (m, 6 H); 2,32 (m, 3 H); 2,40 (m, 3 H); 3,13-3,23 (m, 1 H); 3,76 (s, 3 H); 6,01 (m, 1 H, Hₐᵣₒₘ); 6,77 (m, 2 H, Hₐᵣₒₘ); 7,24 (m, 1 H, Hₐᵣₒₘ); 7,35-7,39 (m, 1 H, Hₐᵣₒₘ); 7,41-7,51 (m, 8 H, Hₐᵣₒₘ); 7,60-7,65 (m, 4 H, Hₐᵣₒₘ) ppm.

¹³C-NMR (CD₂Cl₂): 19,3; 21,6; 23,5; 23,6; 29,3; 56,1; 112,7; 121,3; 123,2; 125,0; 126,0; 127,8; 128,6; 129,2; 129,8; 130,7; 131,2; 133,0 (d, *J*_{CP}= 3,9 Hz); 134,9; 135,6; 136,5; 136,7; 139,0; 143,8; 146,6 (d, *J*_{CP}= 5,4 Hz); 147,4 (d, *J*_{CP}= 8,0 Hz); 152,0 (d, *J*_{CP}= 2,2 Hz) ppm. ESI-TOF/HRMS: m/e 561,21946 (M+H)⁺.

### 6-([1,1':3',1"-Terphenyl]-2'-yloxy)-4-methoxy-2,10-dimethyldibenzo[d,f][1,3,2]dioxaphosphepin.

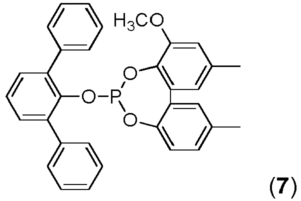

Eine gerührte Lösung von 3-Methoxy-5,5'-dimethyl-[1,1'-biphenyl]-2,2'-diol (0,465 g; 1,903 mmol) in Toluol (12 ml) wurde mit Triethylamin (1,764 g; 17,433 mmol) versetzt und auf 0 °C gekühlt. Zu dieser Mischung wurde eine Lösung von 2,6-Diphenylphenoxydichlorphosphin (0,661 g; 1,903 mmol) in Toluol (3 ml) tropfenweise zugegeben. Die Reaktionsmischung wurde über Nacht gerührt und dann filtriert. Das Filtrat wurde im Vakuum bis zur Trockne eingeengt und der erhaltene Feststoff aus Hexan (20 ml) umkristallisiert. Ausbeute: 0,804 g (1,550 mmol; 82 %).
Elementaranalyse (berechnet für C₃₃H₂₇O₄P = 518,55 g/ mol) C 76,38 (76,44); H 5,18 (5,25); P 5,97 (5,97) %.
³¹P-NMR (CD₂Cl₂): 144,9 ppm.
¹H-NMR (CD₂Cl₂): (m, 6 H); 3,75 (s, 3 H); 5,86 (m, 1 H, Hₐᵣₒₘ); 6,76 (m, 2 H, Hₐᵣₒₘ); 6,92 (m, 1 H, Hₐᵣₒₘ); 7,19 (m, 1 H, Hₐᵣₒₘ); 7,36 (m, 1 H, Hₐᵣₒₘ); 7,40-7,55 (m, 8 H, Hₐᵣₒₘ); 7,60-7,68 (m, 4 H, Hₐᵣₒₘ) ppm.
¹³C-NMR (CD₂Cl₂): 20,9; 21,6; 56,0; 112,9; 121,4; 121,8; 125,1; 127,7; 128,8; 129,9; 130,1; 130,7; 131,3; 132,7 (d, *J*_{CP}= 3,5 Hz); 134,4; 135,1; 135,7; 136,4 (d, *J*_{CP}= 4,4 Hz); 139,0; 146,4 (d, *J*_{CP}= 6,8 Hz); 147,6 (d, *J*_{CP}= 7,4Hz); 152,0 (d, *J*_{CP}=2,5 Hz) ppm.
ESI-TOF/HRMS: m/e 519.17235 (M+H)⁺.

### 6-([1,1':3',1"-Terphenyl]-2'-yloxy)-4-methoxy-2,8,10-trimethyldibenzo[d,f][1,3,2]dioxaphosphepin.

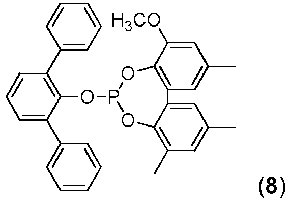

Eine Lösung von 3-Methoxy-3',5,5'-trimethyl-[1,1'-biphenyl]-2,2'-diol (0,492 g; 1,903 mmol) in Toluol (18 ml) wurde mit Triethylamin (1,764 g; 17,435 mmol) versetzt und auf 0 °C gekühlt. Zu dieser Mischung wurde eine Lösung von 2,6-Diphenylphenoxydichlorphosphin (0,727 g; 2,094 mmol) in Toluol (3 ml) tropfenweise zugegeben. Die Reaktionsmischung wurde über Nacht gerührt und dann filtriert. Das Filtrat wurde im Vakuum bis zur Trockne eingeengt, der erhaltene Feststoff bei 50°C / 0,1 mbar getrocknet und durch Säulenchromatografie gereinigt (Hexan/Dichlormethan, 1:1, R*_{f}* = 0,5). Ausbeute: 0,946 g (1,776 mmol; 81 %). Elementaranalyse (berechnet für C₃₄H₂₉O₄P = 532,57 g/ mol) C 76,56 (76,68); H 5,44 (5,49); P 5,95 (5,82) %.
³¹P-NMR (CD₂Cl₂): 145,6 ppm.
¹H-NMR (CD₂Cl₂): (m, 3 H); 2,42 (m, 6 H); 3,84 (s, 3 H); 6,77 (m, 2 H, Hₐᵣₒₘ); 7,03 (m, 2 H, Hₐᵣₒₘ); 7,29-7,34 (m, 5 H, Hₐᵣₒₘ); 7,34-7,37 (m, 1 H, Hₐᵣₒₘ); 7,37-7,40 (m, 1 H, Hₐᵣₒₘ); 7,44 (m, 1 H, Hₐᵣₒₘ); 7,47 (m, 1 H, Hₐᵣₒₘ); 7,55-7,62 (m, 4 H, Hₐᵣₒₘ) ppm.
¹³C-NMR (CD₂Cl₂): 17,0; 21,0; 21,6; 56,3; 112,8; 122,0; 125,0; 127,4; 128,2; 128,3; 129,3; 129,8; 130,3; 130,5; 130,8 (d, *J*_{CP}= 3,3 Hz); 131,0; 131,4; 132,7 (d, *J*_{CP}= 3,6 Hz); 134,2; 135,1; 136,0 (d, *J*_{CP}= 3,1 Hz); 138,9; 139,0; 146,2; 146,3 (d, *J*_{CP}= 6,9Hz); 151,7 (d, *J*_{CP}= 2,0 Hz) ppm. ESI-TOF/HRMS: m/e 533,18778 (M+H)⁺.

### 6-([1,1':3',1"-Terphenyl]-2'-yloxy)-8-methoxy-2,3,10-trimethyldibenzo[d,f][1,3,2]dioxaphosphepin.

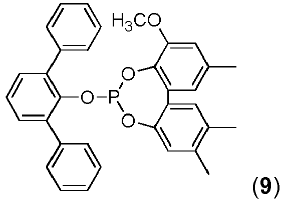

Eine gerührte Suspension von 3-Methoxy-4',5,5'-trimethyl-[1,1'-biphenyl]-2,2'-diol (0,492 g; 1,903 mmol) in Toluol (18 ml) wurde mit Triethylamin (1,764 g; 17,435 mmol) versetzt und auf 0 °C gekühlt. Zu dieser Mischung wurde eine Lösung von 2,6-Diphenylphenoxydichlorphosphin (0,727 g; 2,094 mmol) in Toluol (3 ml) tropfenweise zugegeben. Die Reaktionsmischung wurde über Nacht gerührt und dann filtriert. Das Filtrat wurde im Vakuum bis zur Trockne eingeengt, der erhaltene Feststoff bei 50°C / 0,1 mbar getrocknet und durch Säulenchromatografie gereinigt (Hexan/Dichlormethan, 1:2, R*_{f}* = 0,3). Alternativ wurde die Substanz aus Hexan (23 ml) umkristallisiert. Ausbeute: 0,749 g (1,406 mmol; 74 %).
Elementaranalyse (berechnet für C₃₄H₂₉O₄P = 532,57 g/ mol) C 76,56 (76,68); H 5,31 (5,49); P 5,90 (5,82) %.
³¹P-NMR (CD₂Cl₂): 144,0 ppm.
¹H-NMR (CD₂Cl₂): (m, 3 H); 2,33 (m, 3 H); 2,41 (m, 3 H); 3,77 (s, 3 H); 6,02 (m, 1 H, Hₐᵣₒₘ); 6,78 (m, 2 H, Hₐᵣₒₘ); 7,18 (m, 1 H, Hₐᵣₒₘ); 7,35-7,41 (m, 1 H, Hₐᵣₒₘ); 7,42-7,55 (m, 8 H, Hₐᵣₒₘ); 7,64-7,70 (m, 4 H, Hₐᵣₒₘ) ppm.
¹³C-NMR (CD₂Cl₂): 19,3; 19,9; 21,6; 56,1; 112,7; 121,4; 122,8; 125,1; 127,5 (d, *J*_{CP}= 2,7 Hz); 127,8; 128,6; 130,4; 130,5; 130,7; 131,3; 132,8 (d, *J*_{CP}= 3,8 Hz); 133,4; 135,1; 135,6; 136,6 (d, *J*_{CP}= 4,2 Hz); 138,1; 139,0; 146,6 (d, *J*_{CP}= 5,7 Hz); 147,8 (d, *J*_{CP}= 8,1 Hz); 152,0 (d, *J*_{CP}= 2,0 Hz) ppm.
ESI-TOF/HRMS: m/e 533,18789 (M+H)⁺.

### Arbeitsvorschrift für die Katalyseversuche

Die Hydroformylierung wurde in einem mit Druckkonstanthaltung, Gasflussmessung, Begasungsrührer und Druckpipette ausgestatteten 200 ml-Autoklaven der Fa. Premex Reactor AG, Lengau, Schweiz, durchgeführt. Zur Minimierung eines Einflusses von Feuchtigkeit und Sauerstoff wurde das als Solvens benutzte Toluol mit Natrium-Ketyl getrocknet und unter Argon destilliert. Als Olefin wurde ein Octengemisch eingesetzt: n-Octene (Oxeno GmbH, Octenisomerengemisch aus 1-Octen: ~3 %; *cis*+*trans*-2-Octen: ~49%; *cis*+*trans-3*-Octen: ~29%; *cis*+*trans*-Octen-4: ~16%; gerüstisomere Octene: ~3 % wurden mehrere Stunden über Natrium am Rückfluß erhitzt und unter Argon destilliert.

Für die Versuche wurden im Autoklaven unter Argonatmosphäre folgende Lösungen des Rhodiums in Form von [(acac)Rh(COD)] (acac=Acetylacetonat-Anion; COD=1,5-Cyclooctadien, Umicore) als Katalysatorvorstufe in Toluol eingefüllt: Für Versuche mit 100 ppm-m Rhodium 10 ml einer 4,31 millimolaren, für 40 bzw. 60 ppm-m die gleiche Menge einer entsprechend verdünnten Lösung. Anschließend wurde die entsprechende Menge der in Toluol gelösten Phosphitverbindung (5 Ligandäquivalente pro Rhodium) zugemischt. Durch Zugabe von weiterem Toluol (Die Gesamtmasse an Toluol wurde bestimmt für die GC-Analyse) wurde das Anfangsvolumen der Katalysatorlösung auf 41,0 ml eingestellt. Die jeweils eingebrachte Masse an Toluol wurde bestimmt. Einwaage n-Octene: 10,70 g (95,35 mmol). Der Autoklav wurde bei einem Gesamtgasdruck (Synthesegas: Linde; H₂ (99,999%) : CO (99,997%) = 1:1) von a) 42 bar für einen Enddruck von 50 bar bzw. b) 12 bar für den Enddruck von 20 bar unter Rühren (1500 U/min) auf die jeweils angegebenen Temperaturen aufgeheizt. Nach Erreichen der Reaktionstemperatur wurde der Synthesegasdruck auf a) 48,5 bar für einen Enddruck von 50 bar bzw. b) 19,5 bar für einen Enddruck von 20 bar erhöht und das Edukt mit einem in der Druckpipette eingestellten Überdruck von ca. 3 bar zugepresst. Die Reaktion wurde bei konstantem Druck von jeweils 50 bzw. 20 bar (Nachdruckregler der Fa. Bronkhorst, NL) über 4h geführt. Der Autoklav wurde nach Ablauf der Reaktionszeit auf Zimmertemperatur abgekühlt, unter Rühren entspannt und mit Argon gespült. Jeweils 1 ml der Reaktionsmischungen wurden unmittelbar nach Abschalten des Rührers entnommen, mit 5 ml Pentan verdünnt und gaschromatographisch analysiert: HP 5890 Series II plus, PONA, 50 m x 0,2 mm x 0,5 µm, Die quantitative Bestimmung von Restolefin und Aldehyd erfolgte gegen das Lösungsmittel Toluol als internen Standard.

### Katalyseergebnisse

Die Ergebnisse der Katalyseversuche sind in Tabelle 1 und 2 zusammengefasst.

**Tabelle 1:**

| **Ligand** | **p (bar)** | **T (°C)** | **t (h)** | **[Rh] (ppm)** | **L/Rh** | **Ausb. (%)** |
|---|---|---|---|---|---|---|
| **2** | 20 | 120 | 4 | 100 | 5 | 29 |
| **3** | 20 | 120 | 4 | 100 | 5 | 21 |
| **5*** | 20 | 120 | 4 | 100 | 5 | 88 |
| **6*** | 20 | 120 | 4 | 100 | 5 | 95 |
| **7*** | 20 | 120 | 4 | 100 | 5 | 93 |
| **8*** | 20 | 120 | 4 | 100 | 5 | 91 |
| **9*** | 20 | 120 | 4 | 100 | 5 | 97 |

| | | | | | | |
|---|---|---|---|---|---|---|
| * erfindungsgemäße Verbindung Olefin: n-Octene (Oxeno GmbH) Solvens: Toluol Verhältnis Ligand/Rhodium (L/Rh): 5 : 1 | | | | | | |

Wie aus Tabelle 1 ersichtlich ist, konnte mit den erfindungsgemäßen Verbindungen eine deutlich bessere Ausbeute erzielt werden, als mit den Vergleichsverbindungen 2 und 3.

**Tabelle 2:**

| **Ligand** | **p (bar)** | **T (°C)** | **t (h)** | **[Rh] (ppm)** | **L/Rh** | **Ausb. (%)** |
|---|---|---|---|---|---|---|
| **1** | 50 | 120 | 4 | 100 | 5 | 91 |
| **2** | 50 | 120 | 4 | 100 | 5 | 93 |
| **7*** | 50 | 120 | 4 | 100 | 5 | 95 |

| | | | | | | |
|---|---|---|---|---|---|---|
| * erfindungsgemäße Verbindung Olefin: n-Octene (Oxeno GmbH) Solvens: Toluol Verhältnis Ligand/Rhodium (L/Rh): 5 : 1 | | | | | | |

Auch bei dem erhöhten Druck von 50 bar konnte mit der erfindungsgemäßen Verbindung eine deutlich bessere Ausbeute erzielt werden, als mit den Vergleichsverbindungen 1 und 2.

Anhand der oben beschriebenen Versuche konnte gezeigt werden, dass die gestellte Aufgabe durch die erfindungsgemäßen Verbindungen gelöst wird.

## Patentansprüche

1. Verbindung, welche die allgemeinen Strukturen **I** aufweist: 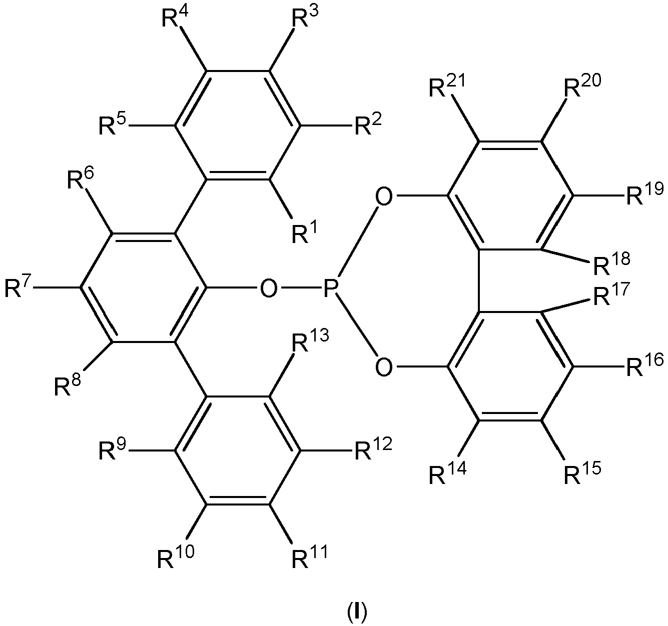 wobei
R¹, R², R³, R⁴, R⁵, R⁶, R⁷, R⁸, R⁹, R¹⁰, R¹¹, R¹², R¹³ ausgewählt sind aus:
-H, -(C₁-C₁₂)-Alkyl, -O-(C₁-C₁₂)-Alkyl, -O-(C₆-C₂₀)-Aryl, -(C₆-C₂₀)-Aryl, -S-Alkyl, -S-Aryl, Halogen, COO-(C₁-C₁₂)-Alkyl, CONH-(C₁-C₁₂)-Alkyl, , -CO-(C₁-C₁₂)-Alkyl, -CO-(C₆-C₂₀)-Aryl, -COOH, -OH, -SO₃H, -CN, -NH₂, -N[(C₁-C₁₂)-Alkyl]₂;
R¹⁴, R¹⁵, R¹⁶, R¹⁷, R¹⁸, R¹⁹, R²⁰, R²¹ ausgewählt sind aus:
-H, -(C₁-C₁₂)-Alkyl, -O-(C₁-C₁₂)-Alkyl, -O-(C₆-C₂₀)-Aryl, -(C₆-C₂₀)-Aryl, -S-Alkyl, -S-Aryl, Halogen, COO-(C₁-C₁₂)-Alkyl, CONH-(C₁-C₁₂)-Alkyl, -CO-(C₁-C₁₂)-Alkyl, -CO-(C₆-C₂₀)-Aryl, -COOH, -OH, - SO₃H, -NH₂, -N[(C₁-C₁₂)-Alkyl]₂;
wobei die genannten Alkyl- und Arylgruppen wie folgt substituiert sein können:
substituierte -(C₁-C₁₂)-Alkylgruppen und substituierte -(C₁-C₁₂)-Alkoxygruppen können in Abhängigkeit von ihrer Kettenlänge, einen oder mehrere Substituenten aufweisen; die Substituenten sind unabhängig voneinander ausgewählt unter -(C₃-C₁₂)-Cycloalkyl, -(C₃-C₁₂)-Heterocycloalkyl, -(C₆-C₂₀)-Aryl, Fluor, Chlor, Cyano, Formyl, Acyl oder Alkoxycarbonyl,
substituierte -(C₆-C₂₀)-Arylgruppen und -(C₆-C₂₀)-Aryl-(C₆-C₂₀)-Arylgruppen können, in Abhängigkeit von der Ringgröße, einen oder mehrere Substituenten aufweisen; diese Substituenten sind unabhängig voneinander ausgewählt unter -H, -(C₁-C₁₂)-Alkyl, -O-(C₁-C₁₂)-Alkyl, -O-(C₆-C₂₀)-Aryl, -(C₆-C₂₀)-Aryl, -Halogen, -COO-(C₁-C₁₂)-Alkyl, -CONH-(C₁-C₁₂)-Alkyl, -(C₆-C₂₀)-Aryl-CON[(C₁-C₁₂)-Alkyl]₂, -CO-(C₁-C₁₂)-Alkyl, -CO-(C₆-C₂₀)-Aryl, -COOH, -OH, -SO₃H; - SO₃Na -NO₂, -CN, -NH₂, -N[(C₁-C₁₂)-Alkyl]₂,
und die beiden Reste mindestens eines der vier folgenden Restepaare nicht für den gleichen Rest stehen: R¹⁴ und R²¹, R¹⁵ und R²⁰, R¹⁶ und R¹⁸, R¹⁷ und R¹⁸.

2. Verbindung nach Anspruch 1,
wobei R¹, R², R³, R⁴, R⁵, R⁶, R⁷, R⁸, R⁹, R¹⁰, R¹¹, R¹², R¹³ ausgewählt sind aus:
-H, -(C₁-C₁₂)-Alkyl, -O-(C₁-C₁₂)-Alkyl, -O-(C₆-C₂₀)-Aryl.

3. Verbindung nach einem der Ansprüche 1 oder 2,
wobei R¹⁴, R¹⁵, R¹⁶, R¹⁷, R¹⁸, R¹⁹, R²⁰, R²¹ ausgewählt sind aus:
-H, -(C₁-C₁₂)-Alkyl, -O-(C₁-C₁₂)-Alkyl, -O-(C₆-C₂₀)-Aryl.

4. Verbindung nach einem der Ansprüche 1 bis 3,
wobei R¹⁴ und R²¹ nicht für den gleichen Rest stehen.

5. Verbindung nach einem der Ansprüche 1 bis 4,
wobei R¹⁵ und R²⁰ nicht für den gleichen Rest stehen.

6. Verbindung nach einem der Ansprüche 1 bis 5,
wobei R¹⁶ und R¹⁹ nicht für den gleichen Rest stehen.

7. Komplex umfassend:
- eine Verbindung nach einem der Ansprüche 1 bis 6,
- ein Metallatom ausgewählt aus: Rh, Ru, Co, Ir.

8. Verwendung einer Verbindung nach einem der Ansprüche 1 bis 6,
zur Katalyse einer Hydroformylierungsreaktion.

9. Verfahren umfassend die Verfahrensschritte:
a) Vorlegen eines Olefins,
b) Zugabe eines Komplexes nach Anspruch 7,
oder einer Verbindung nach einem der Ansprüche 1 bis 6 und einer Substanz, welche ein Metallatom ausgewählt aus: Rh, Ru, Co, Ir aufweist,
c) Zuführen von H₂ und CO,
d) Erwärmen des Reaktionsgemisches, wobei das Olefin zu einem Aldehyd umgesetzt wird.

## Claims

1. Compound having the general structures I: 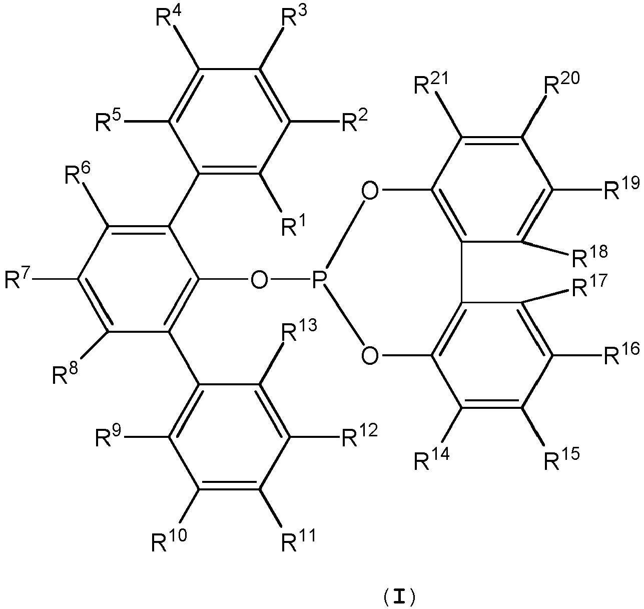 where
R¹, R², R³, R⁴, R⁵, R⁶, R⁷, R⁸, R⁹, R¹⁰, R¹¹, R¹², R¹³ are selected from:
-H, -(C₁-C₁₂)-alkyl, -O-(C₁-C₁₂)-alkyl, -O-(C₆-C₂₀)-aryl, -(C₆-C₂₀)-aryl, -S-alkyl, -S-aryl, halogen, COO-(C₁-C₁₂)-alkyl, CONH-(C₁-C₁₂)-alkyl, -CO-(C₁-C₁₂)-alkyl, -CO-(C₆-C₂₀)-aryl, -COOH, -OH, -SO₃H, -CN, -NH₂, -N[(C₁-C₁₂)-alkyl]₂;
R¹⁴, R¹⁵, R¹⁶, R¹⁷, R¹⁸, R¹⁹, R²⁰, R²¹ are selected from:
-H, -(C₁-C₁₂)-alkyl, -O-(C₁-C₁₂)-alkyl, -O-(C₆-C₂₀)-aryl, -(C₆-C₂₀)-aryl, -S-alkyl, -S-aryl, halogen, COO-(C₁-C₁₂)-alkyl, CONH-(C₁-C₁₂)-alkyl, -CO-(C₁-C₁₂)-alkyl, -CO-(C₆-C₂₀) -aryl, -COOH, -OH, -SO₃H, -NH₂, -N [(C₁-C₁₂)-alkyl]₂;
where the alkyl and aryl groups mentioned may be substituted as follows:
substituted -(C₁-C₁₂)-alkyl groups and substituted -(C₁-C₁₂)-alkoxy groups may have one or more substituents depending on their chain length; the substituents are mutually independently selected from - (C₃-C₁₂)-cycloalkyl, - (C₃-C₁₂)-heterocycloalkyl, - (C₆-C₂₀)-aryl, fluorine, chlorine, cyano, formyl, acyl or alkoxycarbonyl,
substituted -(C₆-C₂₀)-aryl groups and - (C₆-C₂₀)-aryl- (C₆-C₂₀)-aryl groups may have one or more substituents depending on their ring size; these substituents are mutually independently selected from -H, -(C₁-C₁₂)-alkyl, -O-(C₁-C₁₂)-alkyl, -O-(C₆-C₂₀)-aryl, -(C₆-C₂₀) aryl, -halogen, -COO-(C₁-C₁₂) -alkyl, -CONH-(C₁-C₁₂)-alkyl, - (C₆-C₂₀)-aryl-CON[(C₁-C₁₂)-alkyl]₂, -CO-(C₁-C₁₂)-alkyl, -CO-(C₆-C₂₀)-aryl, -COOH, -OH, -SO₃H; -SO₃Na, - NO₂, -CN, -NH₂, -N(C₁-C₁₂)-alkyl]₂,
and the two radicals of at least one of the following four pairs of radicals are not the same radical: R¹⁴, and R²¹, R¹⁵, and R²⁰, R¹⁶ and R¹⁹, R¹⁷, and R¹⁸.

2. Compound according to Claim 1,
where R¹, R², R³, R⁴, R⁵, R⁶, R⁷, R⁸, R⁹, R¹⁰, R¹¹, R¹², R¹³ are selected from:
-H, -(C₁-C₁₂)-alkyl, -O-(C₁-C₁₂)-alkyl, -O-(C₆-C₂₀)-aryl.

3. Compound according to either of Claims 1 and 2, where R¹⁴, R¹⁵, R¹⁶, R¹⁷, R¹⁸, R¹⁹, R²⁰, R²¹ are selected from:
-H, -(C₁-C₁₂)-alkyl, -O-(C₁-C₁₂)-alkyl, -0- (C₆-C₂₀)-aryl.

4. Compound according to any of Claims 1 to 3,
where R¹⁴, and R²¹ are not the same radical.

5. Compound according to any of Claims 1 to 4,
where R¹⁵ and R²⁰ are not the same radical.

6. Compound according to any of Claims 1 to 5,
where R¹⁶ and R¹⁹ are not the same radical.

7. Complex comprising:
- a compound according to any of Claims 1 to 6,
- a metal atom selected from: Rh, Ru, Co, Ir.

8. Use of a compound according to any of Claims 1 to 6, for catalyzing a hydroformylation reaction.

9. A process comprising the following process steps:
a) initially charging an olefin,
b) adding a complex according to Claim 7,
or a compound according to any of Claims 1 to 6 and a substance having a metal atom selected from: Rh, Ru, Co, Ir.
c) feeding in H₂ and CO,
d) heating the reaction mixture, with conversion of the olefin to an aldehyde.

## Revendications

1. Composé qui présente la structure générale I : 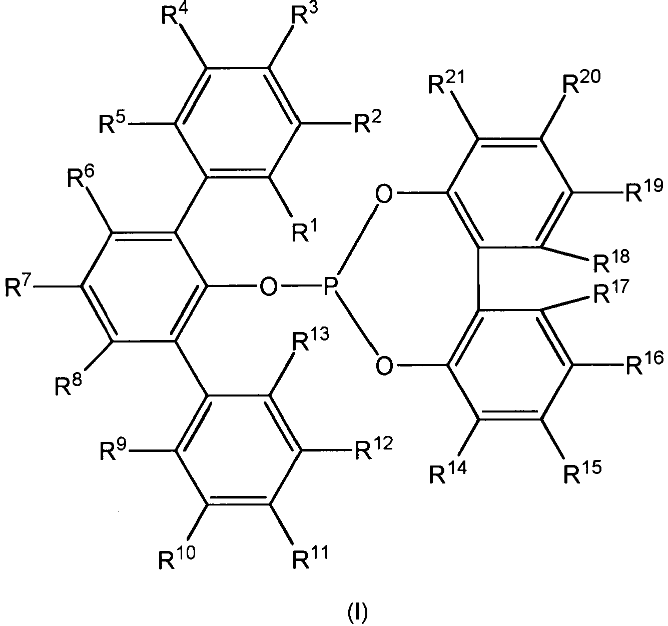
dans laquelle R¹, R², R³, R⁴, R⁵, R⁶, R⁷, R⁸, R⁹, R¹⁰, R¹¹, R¹², R¹³ sont choisis parmi :
-H, - (C₁-C₁₂)-alkyle, -O- (C₁-C₁₂)-alkyle, -O-(C₆-C₂₀)-aryle, - (C₆-C₂₀)-aryle, -S-alkyle, -S-aryle, halogène, COO-(C₁-C₁₂)-alkyle, CONH-(C₁-C₁₂)-alkyle, -CO-(C₁-C₁₂)-alkyle, -CO-(C₆-C₂₀)-aryle, -COOH, -OH, -SO₃H, -CN, -NH₂, -N[(C₁-C₁₂)-alkyle]₂ ; R¹⁴, R¹⁵, R¹⁶, R¹⁷, R¹⁸, R¹⁹, R²⁰, R²¹ sont choisis parmi :
-H, - (C₁-C₁₂)-alkyle, -O-(C₁-C₁₂)-alkyle, -O-(C₆-C₂₀)-aryle, -(C₆-C₂₀)-aryle, -S-alkyle, -S-aryle, halogène, COO-(C₁-C₁₂)-alkyle, CONH-(C₁-C₁₂)-alkyle, -CO-(C₁-C₁₂)-alkyle, -CO-(C₆-C₂₀)-aryle, -COOH, -OH, -SO₃H, -NH₂, -N[(C₁-C₁₂)-alkyle]₂ ;
les groupes alkyle et aryle mentionnés pouvant être substitués comme suit :
les groupes - (C₁-C₁₂) -alkyle substitués et les groupes -(C₁-C₁₂)-alcoxy substitués peuvent présenter, en fonction de leur longueur de chaîne, un ou plusieurs substituants ; les substituants sont choisis, indépendamment les uns des autres, parmi -(C₃-C₁₂)-cycloalkyle, - (C₃-C₁₂)-hétérocycloalkyle, -(C₆-C₂₀)-aryle, fluor, chlore, cyano, formyle, acyle ou alcoxycarbonyle, les groupes - (C₆-C₂₀)-aryle substitués et les groupes - (C₆-C₂₀)-aryl-(C₆-C₂₀)-aryle substitués peuvent présenter, en fonction de leur dimension de cycle, un ou plusieurs substituants ; ces substituants sont choisis, indépendamment les uns des autres, parmi -H, -(C₁-C₁₂)-alkyle, -O-(C₁-C₁₂)-alkyle, -O-(C₆-C₂₀)-aryle, - (C₆-C₂₀)-aryle, -halogène, -COO-(C₁-C₁₂)-alkyle, -CONH-(C₁-C₁₂)-alkyle, -(C₆-C₂₀)-aryl-CON[(C₁-C₁₂)-alkyle]₂, -CO-(C₁-C₁₂)-alkyle, -CO-(C₆-C₂₀)-aryle, -COOH, -OH, -SO₃H, -SO₃Na, -NO₂, -CN, -NH₂, -N[(C₁-C₁₂)-alkyle]₂ ;
et les deux radicaux d'au moins une des quatre paires de radicaux suivantes ne représentent pas le même radical : R¹⁴, et R²¹, R¹⁵ et R²⁰, R¹⁶ et R¹⁹, R¹⁷ et R¹⁸.

2. Composé selon la revendication 1, R¹, R², R³, R⁴, R⁵, R⁶, R⁷, R⁸, R⁹, R¹⁰, R¹¹, R¹², R¹³ étant choisis parmi : -H, -(C₁-C₁₂)-alkyle, -O-(C₁-C₁₂)-alkyle, -O-(C₆-C₂₀)-aryle.

3. Composé selon l'une quelconque des revendications 1 ou 2, R¹⁴, R¹⁵, R¹⁶, R¹⁷, R¹⁸, R¹⁹, R²⁰, R²¹ étant choisis parmi :
-H, -(C₁-C₁₂)-alkyle, -O-(C₁-C₁₂)-alkyle, -O-(C₆-C₂₀)-aryle.

4. Composé selon l'une quelconque des revendications 1 à 3, R¹⁴, et R²¹ ne représentant pas le même radical.

5. Composé selon l'une quelconque des revendications 1 à 4, R¹⁵, et R²⁰ ne représentant pas le même radical.

6. Composé selon l'une quelconque des revendications 1 à 5, R¹⁶ et R¹⁹ ne représentant pas le même radical.

7. Complexe, comprenant :
- un composé selon l'une quelconque des revendications 1 à 6,
- un atome métallique choisi parmi : Rh, Ru, Co, Ir.

8. Utilisation d'un composé selon l'une quelconque des revendications 1 à 6 pour la catalyse d'une réaction d'hydroformylation.

9. Procédé comprenant les étapes de procédé :
a) disposition préalable d'une oléfine,
b) addition d'un complexe selon la revendication 7 ou d'un composé selon l'une quelconque des revendications 1 à 6 et d'une substance qui présente un atome métallique choisi parmi : Rh, Ru, Co, Ir,
c) introduction de H₂ et de CO,
d) chauffage du mélange réactionnel, l'oléfine étant transformée en aldéhyde.
